# EUROPEAN PATENT SPECIFICATION

(11) **EP 1 992 949 B1**
(45) Date of publication and mention of the grant of the patent: **19.09.2018**
(21) Application number: 07738266.1
(22) Date of filing: 12.03.2007
(51) Int. Cl.: H01L 21/762, H01L 21/84

(54) **METHOD FOR MANUFACTURING SOI WAFER**
VERFAHREN ZUR HERSTELLUNG VON SOI-WAFERN
PROCEDE DE FABRICATION DE PLAQUETTE SOI

(30) Priority: 13.03.2006 JP 2006067804
(43) Date of publication of application: 19.11.2008
(73) Proprietor: Shin-Etsu Chemical Co., Ltd., Tokyo 100-0004 (JP)
(72) Inventor: AKIYAMA, Shoji, Annaka-shi, Gunma 3790195 (JP); KUBOTA, Yoshihiro, Annaka-shi, Gunma 3790195 (JP); ITO, Atsuo, Annaka-shi, Gunma 3790195 (JP); TANAKA, Koichi, Annaka-shi, Gunma 3790195 (JP); KAWAI, Makoto, Annaka-shi, Gunma 3790195 (JP); TOBISAKA, Yuuji, Annaka-shi, Gunma 3790195 (JP)
(74) Representative: Wibbelmann, Jobst
(86) International application number: PCT/JP2007/054794
(87) International publication number: WO 2007/105676

(56) References cited:
- WO-A1-00/63965
- WO-A2-2006/023289
- JP-A- 11 145 438
- JP-A- 52 111 128
- JP-A- 2002 350 347
- JP-A- 2004 101 253
- JP-A- 2005 005 708
- JP-A- 2005 024 286
- JP-A- 2005 294 800
- US-A1- 2003 116 802
- US-A1- 2004 229 444

## Description

### Technical Field

The present invention relates to a method of manufacturing an SOI substrate.

### Background Art

In recent years, attention has been drawn to a small biochip used to efficiently analyze small amounts of sample in a short period of time. Such a microchip as described above is generally obtained by fabricating a pattern and the like having a width of several tens to several hundreds of micrometers and a depth of several to several tens of micrometers onto a substrate, such as a glass substrate, using a photolithographic technique heretofore known as semiconductor technology. This microchip is expected to be applied to fields referred to as µ-TAS (Micro-Total Analysis Systems), LOAC (Lab-On-A Chip), Bio-MEMS (Bio-Micro Electro-Mechanical Systems), Optical-MEMS, Fluidic-MEMS, and the like.

In the conventional structure of these microchips, however, there is usually provided only a portion to be figuratively referred to as a "chemical plant," in which individual microfabricated parts intended, for example, to flow or retain a measurement sample (mostly solution) or cause a chemical reaction therein, are integrated and provided on a single chip (as the substrate of which a transparent material such as quartz is used). Hence, semiconductor elements and the like necessary for the analysis and evaluation of the measurement sample are mounted on another device separately from this microchip, thus impeding the implementation of simple, highly efficient analysis and evaluation.

In order to overcome such an impediment, there arises the need for an integrated microchip in which the "chemical plant" portion and semiconductor elements and the like necessary for the analysis and evaluation of the measurement sample are mounted on a single chip. To take a bench-top biochip as an example, a substrate transparent to incident light and a high-quality semiconductor layer for forming semiconductor elements on this transparent substrate are necessary in order to take out an electrical signal by injecting light into the measurement sample. In order to meet such needs as described above, there has been proposed using an SOS (Silicon on Sapphire) substrate which is a type of SOI substrate (see Hidekazu Uchida et al., "Characteristic Improvement of Surface Photovoltage Method-Based Two-Dimensional Chemical Image Sensor Using SOS Substrate," The Institute of Electrical Engineers of Japan, Chemical Sensor Workshop Material CHS-00-66 (2000) 23).

US2004/229444 describes a method for production of a semiconductor-on-insulator (SOI) structure. The method comprises providing first and second substrates, where first substrate comprises internal zone for separating first substrate into first and second parts, and second substrate comprises oxide glass or oxide glass-ceramic; bringing bonding surfaces into contact; and cooling the bonded first and second substrates and separating the first and second parts at the separation zone.

WO 2006/023289 relates to semiconductor-on-insulator structures having strained semiconductor layers. According to one embodiment of the invention, a semiconductor-on-insulator structure has a first layer including a semiconductor material, attached to a second layer including a glass or glass-ceramic, with the CTEs of the semiconductor and glass or glass-ceramic selected such that the first layer is under tensile strain.

However, since an SOS substrate is obtained by heteroepitaxially growing a silicon layer on a sapphire substrate and, therefore, a high-density dislocation (lattice defect) occurs at a boundary face between silicon and sapphire due to a difference in lattice constant therebetween, it is not easy to enhance the quality of the silicon layer. In addition, there has been pointed out the problem that since the sapphire substrate itself is costly, the SOS substrate unavoidably tends to be also expensive.

Incidentally, as one of methods for obtaining an SOI substrate, there is known the SmartCut method based on the bonding together of substrates. The SmartCut method is a method in which a silicon substrate, on the bonding surface side of which hydrogen ions have been implanted, and a substrate made also of silicon or of another material are bonded together and subjected to a relatively high-temperature heat treatment. Then, a silicon thin film is thermally peeled off from a region where the concentration of the implanted hydrogen ions is highest, thus obtaining an SOI substrate (see, for example, Japanese Patent No. 3048201 and A. J. Auberton-Herve et al., "SMART CUT TECHNOLOGY: INDUSTRIAL STATUS of SOI WAFER PRODUCTION and NEW MATERIAL DEVELOPMENTS" (Electrochemical Society Proceedings Volume 99-3 (1999) pp. 93-106)).

In a case where a silicon substrate and a glass substrate are selected as substrates to be bonded together, however, the substrates are more likely to cause breakage or local cracks if the temperature of heat treatment applied to the substrates being bonded in a manufacturing process becomes higher, since the two substrates differ in thermal properties (for example, thermal expansion rate and intrinsic allowable temperature limits) from each other. From this point of view, the SmartCut method which requires high temperatures for silicon thin film separation can hardly be said preferable as a method for manufacturing an SOI substrate based on the bonding of a silicon substrate to a glass substrate.

The present invention has been accomplished in view of the above-described problems. It is therefore an object of the present invention to avoid the introduction of breakage, local cracks and the like due to a difference in thermal properties between a silicon substrate and a glass substrate, thereby providing an SOI substrate having an SOI layer superior in film uniformity, crystal quality, and electrical characteristics (carrier mobility and the like), as well as providing, using this SOI substrate, a microchip (biochip) in which a hole, a micro-flow passage or the like and a semiconductor element for analysis and evaluation are integrated into a single chip, or a macro chip, such as a surface potential sensor, capable of monitoring a change in the charge amount of a sample (for example, cell) from a detected photocurrent.

### Disclosure of the Invention

In order to solve the above-described problems, the present invention suggests a method of manufacturing an SOI substrate as set forth in greater detail in the claims, and including steps (1) to (4) described below: (1) a step of forming a hydrogen ion-implanted layer by implanting ions into the bonding surface of a silicon substrate; (2) a step of applying a surface activation treatment to the bonding surface of at least one of the silicon substrate and the glass substrate; (3) a step of bonding together the silicon substrate and the glass substrate; and (4) a step of transferring a silicon layer onto the glass substrate by peeling off the surface layer of the silicon substrate along the hydrogen ion-implanted layer at a temperature of 300°C or lower.

The step (2) of surface activation treatment can be carried out by means of at least one of plasma treatment and ozone treatment. In addition, the step (3) includes a sub-step of heat-treating the silicon substrate and the glass substrate after the bonding together, with the two substrates bonded together.

In the present invention, the sub-step of heat treatment is carried out at a temperature of 100°C or higher but not higher than 300°C. In addition, the method may include a step (step (5)) of polishing the peeling plane of the silicon layer, in succession to the step (4), so that the surface roughness (RMS) thereof is not greater than 3 nm.

The microchip of the present invention is, for example, such that one principal surface of the glass substrate has a concave portion, such as a flow passage or a hole, and a semiconductor element for analyzing/evaluating a sample attached/held to the concave portion is provided in the silicon layer provided on the other principal surface of the glass substrate.

Furthermore, the microchip of the present invention includes, for example, an insulating layer formed on a surface of the silicon layer; a sample-holding portion provided on the insulating layer; a biasing portion for forming a depletion layer in a boundary face between the insulating layer and the silicon layer; and a signal-detecting circuit for detecting the amount of photoelectric current generated depending on the thickness of the depletion layer which varies according to the amount of charge provided by an analyte held by the sample-holding portion.

An SOI substrate suitable for the manufacture of a microchip is fabricated by a method including the steps (1) to (4), that is: (1) a step of forming a hydrogen ion-implanted layer by implanting ions into the bonding surface of a silicon substrate; (2) a step of applying a surface activation treatment to the bonding surface of at least one of the silicon substrate and the glass substrate; (3) a step of bonding together the silicon substrate and the glass substrate; and (4) a step of transferring a silicon layer onto the glass substrate by peeling off the surface layer of the silicon substrate along the hydrogen ion-implanted layer at a temperature of 300°C or lower. Note that it is preferable that the above-described glass substrate is a quartz substrate.

And step (3) includes a sub-step of heat-treating said silicon substrate and said glass substrate after said bonding together, with said silicon substrate and said glass substrate bonded together, and wherein said sub-step of heat treatment is carried out at a temperature of 100°C or higher but not higher than 300°C.

Since the present invention has made it possible to fabricate an SOI substrate without applying such high-temperature treatments (for example, approximately 1000°C) as applied in conventional methods, breakage, local cracks and the like due to a difference in thermal properties between the silicon substrate and the glass substrate are avoided. As a result, it is possible to provide an SOI substrate having an SOI layer superior in film uniformity, crystal quality, and electrical characteristics (carrier mobility and the like).

Then, a concave portion, such as a hole, a micro-flow passage or a micromixer is formed on a surface of the glass substrate of the SOI substrate thus obtained and a surface treatment is performed using a silane coupling agent or the like, so that processes required for a DNA chip or a microfluidic chip are applied. In addition, a semiconductor element portion for the analysis/evaluation of a sample attached/held to this concave portion is formed in the SOI layer. Consequently, it is possible to obtain a microchip (biochip) in which a hole, a micro-flow passage or the like and a semiconductor element for analysis/evaluation are integrated into a single chip.

Furthermore, an insulating layer, such as a silicon dioxide film or a silicon nitride film, is formed on a surface of the SOI layer, a sample-holding portion to which a measurement sample is attached or held is provided on this insulating layer, and biasing electrodes used to form a depletion layer in a boundary face between the insulating layer and the SOI layer and a signal-detecting circuit for detecting the amount of photoelectric current generated depending on the thickness of the depletion layer which varies according to the amount of charge provided by an analyte held by the sample-holding portion are further provided. Consequently, it is possible to obtain a macro chip, such as a surface potential sensor, capable of monitoring a change in the charge amount of a sample (for example, cell) from a detected photocurrent.

### Brief Description of the Drawings

Figures 1(A) to 1(H) are schematic views used to explain a manufacturing process example of an SOI substrate of the present invention;
Figures 2(A) to 2(C) are conceptual schematic views used to explain ways of processing for silicon thin film separation;
Figures 3(A) and 3(B) are schematic views used to explain a first constitution of a microchip of the present invention; and
Figure 4 is a schematic view used to explain a second constitution of a microchip of the present invention.

### Best Mode for Carrying Out the Invention

Hereinafter, the best mode for carrying out the present invention will be described with reference to the accompanying drawings. Note that in the following description, a glass substrate is assumed to be a quartz substrate.

### [Substrate for the manufacture of microchips]:

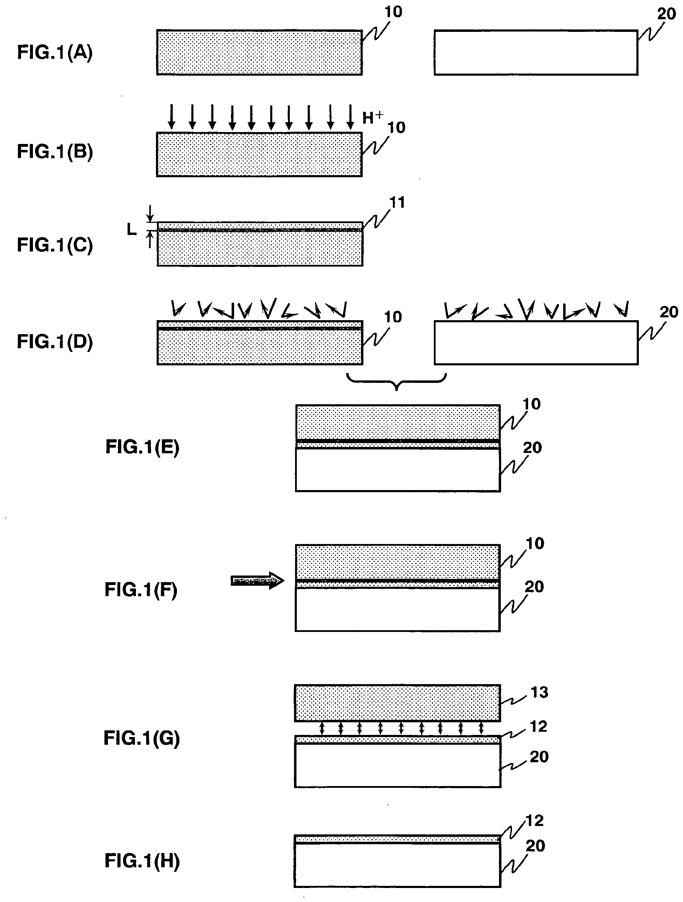

Figures 1(A) to 1(H) are schematic views used to explain a manufacturing process example of the present invention of an SOI substrate, wherein a substrate 10 illustrated in Figure 1(A) is a single-crystal Si substrate and a substrate 20 is a quartz substrate. Here, the single-crystal Si substrate 10 is, for example, a commercially-available Si substrate grown by the CZ method (Czochralski method). The electrical property values, such as the conductivity type and specific resistivity, the crystal orientation, and the crystal diameter of the single-crystal Si substrate 10 are selected as appropriate, depending on the design value and process of a semiconductor element formed on the SOI layer (Si thin film layer) of an SOI substrate manufactured using the method of the present invention or on the area of each individual microchip. In addition, this single-crystal Si substrate 10 may be in a state in which an oxide film has been previously formed on a surface (bonding surface) thereof.

Note that the diameters of these substrates are substantially the same. For the sake of convenience in a subsequent device formation process, it is advantageous to provide the same orientation flat (OF) as the OF provided in the single-crystal Si substrate 10 also in the quartz substrate 20, and bond the substrates together by aligning these OFs with each other.

First, hydrogen ions are implanted into a surface of the single-crystal Si substrate 10 (Figure 1(B)) to form a hydrogen ion-implanted layer on the surface layer of the single-crystal Si substrate 10. This ion-implanted surface serves as a later-discussed bonding surface (joint surface). As the result of this hydrogen ion implantation, a uniform ion-implanted layer 11 is formed near a surface of the single-crystal Si substrate 10 at a predetermined depth (average ion implantation depth L). In a region at a depth corresponding to the average ion implantation depth L in a surface region of the single-crystal Si substrate 10, there is formed a "microbubble layer" which exists locally in the aforementioned region (Figure 1(C)).

The depth of the ion-implanted layer 11 from the surface of the single-crystal Si substrate 10 (average ion implantation depth L) is controlled by an acceleration voltage at the time of ion implantation and is determined depending on how thick an SOI layer to be peeled off is desired. For example, the average ion implantation depth L is set to approximately 2 to 3 µm and the acceleration voltage is set to 50 to 100 keV. Note that an insulating film, such as an oxide film, may be previously formed on the ion-implanted surface of the single-crystal Si substrate 10 and ion implantation may be applied through this insulating film in a process of ion implantation into Si crystal, as is commonly practiced to suppress the channeling of implanted ions.

A plasma treatment or an ozone treatment for the purpose of surface cleaning, surface activation and the like is applied to the respective bonding surfaces of the single-crystal Si substrate 10 in which the ion-implanted layer 11 has been formed and the quartz substrate 20 (Figure 1(D)). Note that such a surface treatment as described above is performed for the purpose of removing organic matter from a surface serving as a bonding surface or achieving surface activation by increasing surface OH groups. However, the surface treatment need not necessarily be applied to both of the bonding surfaces of the single-crystal Si substrate 10 and the quartz substrate 20. Rather, the surface treatment may be applied to either one of the two bonding surfaces.

When carrying out this surface treatment by means of plasma treatment, a surface-cleaned single-crystal Si substrate to which RCA cleaning or the like has been applied previously and/or a quartz substrate is mounted on a sample stage within a vacuum chamber, and a gas for plasma is introduced into the vacuum chamber so that a predetermined degree of vacuum is reached. Note that examples of gas species for plasma used here include an oxygen gas, a hydrogen gas, an argon gas, a mixed gas thereof, or a mixed gas of oxygen and helium for use in the surface treatment of the single-crystal Si substrate. The gas for plasma can be changed as appropriate according to the surface condition of the single-crystal Si substrate or the purpose of use thereof.

If the surface treatment is performed also for the purpose of oxidizing a single-crystal Si surface, a gas containing at least an oxygen gas is used as the gas for plasma. Note that the surface of the quartz substrate is in an oxidized state and, therefore, there are no particular restrictions on such selection of a type of gas for plasma as described above. High-frequency plasma having an electrical power of approximately 100 W is generated after the introduction of the gas for plasma, thereby applying a treatment for approximately 5 to 10 seconds to a surface of the single-crystal Si substrate and/or a surface of the quartz substrate to be plasma-treated, and then finishing the treatment.

When the surface treatment is carried out by means of ozone treatment, a surface-cleaned single-crystal Si substrate to which RCA cleaning or the like has been applied previously and/or a quartz substrate is mounted on a sample stage within a chamber placed in an oxygen-containing atmosphere. Then, after introducing a gas for plasma, such as a nitrogen gas or an argon gas, into the chamber, high-frequency plasma having a predetermined electrical power is generated to convert oxygen in the atmosphere into ozone by the plasma. Thus, a surface treatment is applied for a predetermined length of time to a surface of the single-crystal Si substrate and/or a surface of the quartz substrate to be treated.

The single-crystal Si substrate 10 and the quartz substrate 20, to which such a surface treatment as described above has been applied, are bonded together with the surfaces thereof closely adhered to each other as bonding surfaces (Figure 1(E)). As described above, the surface (bonding surface) of at least one of the single-crystal Si substrate 10 and the quartz substrate 20 has been subjected to a surface treatment by plasma treatment, ozone treatment or the like and is therefore in an activated state. Thus, it is possible to obtain a level of bonding strength fully resistant to mechanical separation or mechanical polishing in a post-process even if the substrates are closely adhered to each other (bonded together) at room temperature. If the substrates need to have an even higher level of bonding strength, there may be provided a sub-step of applying a "bonding process" by heating the substrates at a relatively low temperature in succession to the "bonding together" illustrated in Figure 1(E).

The bonding process temperature at this time is set within a range from 100 to 300°C, taking into consideration the condition that the substrates to be used for bonding are a silicon substrate and a quartz substrate (glass substrate). The reason for selecting such a temperature as described above is because consideration is given to a difference in thermal expansion coefficient between single-crystal Si and quartz, an amount of strain due to this difference, and a relationship between the amount of strain and the thicknesses of the single crystal Si substrate 10 and the quartz substrate 20. If the thicknesses of the single-crystal Si substrate 10 and the quartz substrate 20 are almost the same with each other, thermal strain-induced cracks or separation at a bonding plane occurs due to a difference in rigidity between the two substrates when the substrates are subjected to a heat treatment at a temperature higher than 350°C, since there is a significant difference between the thermal expansion coefficient (2.33 × 10⁻⁶) of single-crystal Si and the thermal expansion coefficient (0.6 × 10⁻⁶) of quartz. In an extreme case, the breakage of the single-crystal Si substrate or the quartz substrate occurs. Accordingly, the upper limit of the heat treatment temperature is specified as 350°C and a heat treatment is preferably applied within a temperature range of 100 to 300°C.

If a force of impact is applied to the bonded substrate using a certain technique in succession to such a treatment as described above (Figure 1(F)), a silicon thin film peels off from the bulk portion 13 of single-crystal silicon along the hydrogen ion-implanted layer 11 due to this impact, thereby obtaining an SOI substrate having an SOI layer 12 on the quartz substrate 20 (Figure 1(H)).

Note here that there can be various ways of externally applying impact in order to peel off the silicon thin film.

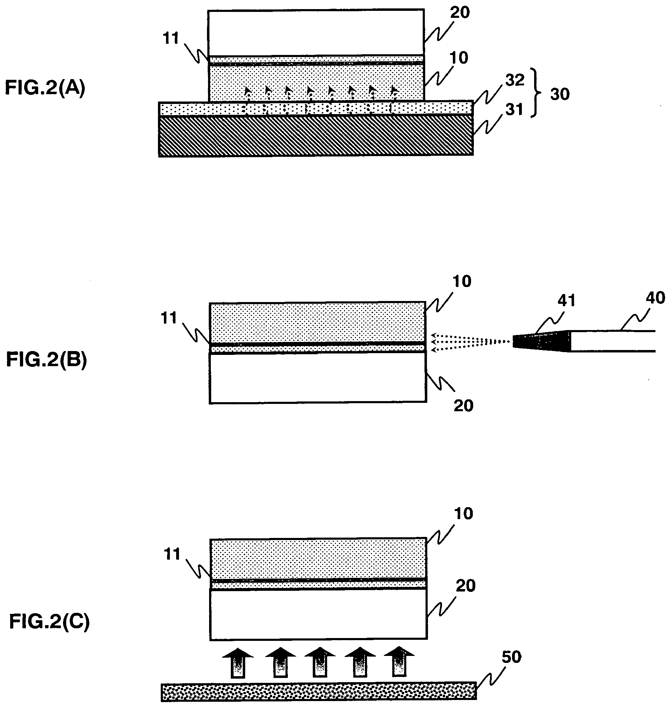

Figures 2(A) to 2(C) are conceptual schematic views used to exemplify various techniques for peeling off a silicon thin film, wherein Figure 2(A) illustrates an example of performing separation by thermal shock, Figure 2(B) illustrates an example of performing separation by mechanical shock, and Figure 2(C) illustrates an example of performing separation by vibratory shock.

In Figure 2(A), reference numeral 30 denotes a heating section. In this figure, a heating plate 32 having a smooth surface is placed on a hot plate 31, and the smooth surface of this heating plate 32 is closely adhered on the rear surface of the single-crystal Si substrate 10 bonded to the quartz substrate 20. Although a dummy silicon substrate is used here as the heating plate 32, there are no particular restrictions on the material of the heating plate as long as a smooth surface is available (semiconductor substrate or ceramic substrate). Silicone rubber or the like can also be used as the heating plate material, though not suited for use at temperatures above 250°C since the allowable temperature limit of the rubber is considered to be approximately 250°C. The heating plate 32 need not be used in particular, as long as the surface of the hot plate 31 is sufficiently smooth. Alternatively, the hot plate 31 itself may be used as the "heating plate."

When the temperature of the heating plate 32 is kept at 300°C or lower (for example, 250 to 300°C) and the rear surface of the single-crystal Si substrate 10 bonded to the quartz substrate 20 is closely adhered on this heating plate 32, the single-crystal Si substrate 10 is heated by thermal conduction, thereby generating a temperature difference between the Si substrate and the quartz substrate 20. As described above, since the thermal expansion coefficient of the silicon substrate is larger than the thermal expansion coefficient of the quartz substrate, a large stress is generated between the two substrates due to the rapid expansion of the single-crystal Si substrate 10 if the single-crystal Si substrate 10 in a bonded state is heated from the rear surface thereof. The separation of a silicon thin film is caused by this stress.

The example illustrated in Figure 2(B) utilizes a jet of a fluid to apply mechanical shock. That is, a fluid, such as a gas or a liquid, is sprayed in a jet-like manner from the leading end 41 of a nozzle 40 at a side surface of the single-crystal Si substrate 10, thereby applying impact. An alternative technique, for example, is to apply impact by pressing the leading end of a blade against a region near the ion-implanted layer 11.

Yet alternatively, as illustrated in Figure 2(C), the separation of a silicon thin film may be caused by applying vibratory shock using ultrasonic waves emitted from the vibrating plate 50 of an ultrasonic oscillator.

Evaluation of the surface condition of an SOI substrate obtained by following such a series of processes as described above showed that there were no defects, such as the local separation of a silicon thin film, traces of separation and untransferred regions. Thus, the substrate surface exhibited an extremely planar state. Measurement of a 10 µm × 10 µm area of the surface of the SOI layer after separation using an atomic force microscope (AFM) showed that the RMS mean value was as excellent as no greater than 5 nm. In addition, the film-thickness variation (PV: peak-to-valley) of the SOI layer within the substrate surface was no larger than 4 nm.

Note that there may be provided a step of polishing the surface of the SOI layer 12 in succession to the step of Figure 1(H), in order to obtain an SOI layer having an even higher degree of planarity (for example, SOI layer having an RMS value of 3 nm or smaller). It is needless to say that when such a polishing step as described above is provided, the depth (average ion implantation depth L) of formation of the hydrogen ion-implanted layer 11 is set by previously allowing for a "machining allowance" to be lost by polishing.

As described above, only low-temperature treatments are applied consistently at 300°C or lower in a manufacturing process of the SOI substrate of the present invention. Whereas a conventional "bonding method" requires high-temperature heat treatments for the purpose of obtaining sufficient bonding strength or breaking silicon atomic bonds (see, for example, Japanese Patent No. 3048201 and Japanese Patent Laid-Open No. 11-145438), the present invention does not require such high-temperature heat treatments (for example, 1000°C or higher). The SOI substrate therefore has an SOI layer having little defects and superior in film uniformity, crystal quality, and electrical characteristics (carrier mobility and the like). Furthermore, according to the above-described process, it is possible to obtain the SOI substrate without causing any breakage, cracks and the like due to a difference in thermal expansion coefficient between the silicon substrate and the quartz substrate since the SOI substrate does not undergo heat treatments at temperatures in excess of 300 to 350°C.

A concave portion, such as a hole, a micro-flow passage or a micromixer, is formed on a surface of the glass substrate of the SOI substrate thus obtained and a surface treatment is performed using a silane coupling agent or the like, so that processes required for a DNA chip or a microfluidic chip are applied. In addition, a semiconductor element portion for the analysis/evaluation of a sample attached/held to this concave portion is formed in the SOI layer. Consequently, it is possible to obtain a microchip (biochip) in which a hole, a micro-flow passage or the like and a semiconductor element for analysis/evaluation are integrated into a single chip.

Furthermore, an insulating layer, such as a silicon dioxide film or a silicon nitride film, is formed on a surface of the SOI layer 12, a sample-holding portion to which a measurement sample is attached or held is provided on this insulating layer, and biasing electrodes used to form a depletion layer in a boundary face between the insulating layer and the SOI layer 12 and a signal-detecting circuit for detecting the amount of photoelectric current generated depending on the thickness of the depletion layer which varies according to the amount of charge provided by an analyte held by the sample-holding portion are further provided. Consequently, it is possible to obtain a macro chip, such as a surface potential sensor, capable of monitoring a change in the charge amount of a sample (for example, cell) from a detected photocurrent.

Hereinafter, constitutional examples of a microchip of the present invention will be described with reference to embodiments thereof.

### Embodiment 1

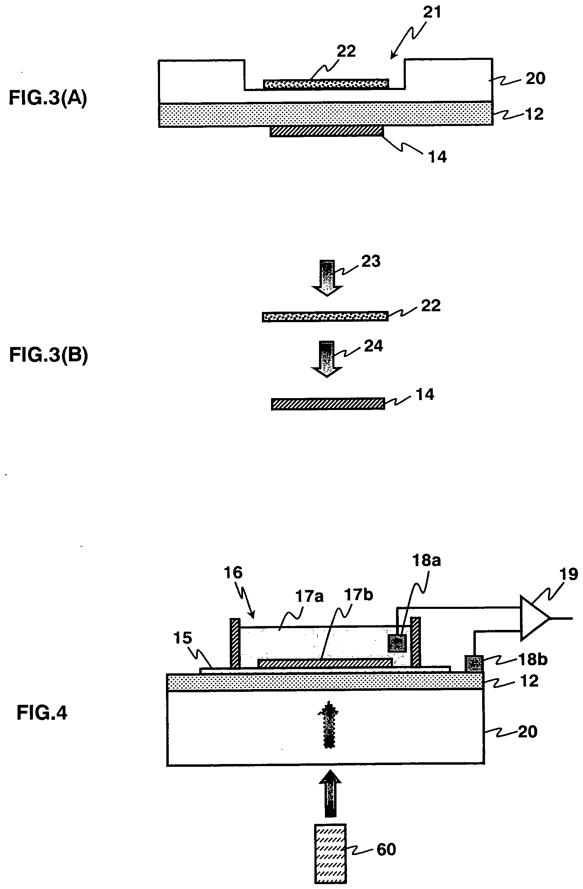

Chip equipped with semiconductor element for fluorescence/absorbed light analysis: Figure 3(A) is a cross-sectional view used to explain a first constitution of a microchip of the present invention, wherein the microchip shown in this figure is a chip equipped with a semiconductor element for analyzing fluorescence and absorbed light from a measurement sample. In this figure, reference numerals 12 and 20 denote an SOI layer and a quartz substrate, respectively, wherein a concave portion 21 is formed on one principal surface of the quartz substrate 20 and a sensitive membrane 22 is provided in this concave portion 21. This sensitive membrane 22 is the measurement sample itself or a membrane to which the measurement sample is attached/held and is, for example, one of DNA, a lipid membrane, an enzyme membrane, an antibody membrane, a nitride film and the like. If the measurement sample is an antibody, an antigen may be previously attached to the concave portion 21. In that case, the antibody serves as the "sensitive membrane."

Although only one concave portion 21 provided with the sensitive membrane 22 is illustrated in Figure 3(A), the concave portion 21 can have various forms and layouts according to the usage of the microchip. For example, a pump, a valve, a micro-flow passage, an injection portion, a reaction portion, a separation portion, and the like are also regarded as the concave portion 21 of the present invention. Note that such a concave portion 21 as described above may be formed before the quartz substrate 20 is bonded to the single-crystal Si substrate 10. In the present embodiment, however, the concave portion 21 is formed on a surface of the quartz substrate 20 after transferring the SOI layer 12 to the quartz substrate 20 so that an SOI substrate is provided.

On the other hand, in a predetermined part of the SOI layer 12, there is formed a semiconductor element portion 14 for analyzing/evaluating a sample (sensitive membrane 22 in the case of the present embodiment) attached/held to the concave portion 21. In the microchip illustrated in Figure 3(A), analysis/evaluation is performed by irradiating light (23) having a wavelength of λ = 1.1 µm or shorter at the measurement sample (22) and detecting fluorescence or absorbed light (24) from the measurement sample (22) using a semiconductor element portion (14) (see Figure 3(B)). The reason for setting the wavelength of probe light to 1.1 µm or shorter is because light having a wavelength longer than this wavelength transmits through a silicon crystal and, therefore, cannot be detected by the semiconductor element portion 14.

In the semiconductor element portion 14, there are provided a light-receiving element for receiving fluorescence or absorbed light from the measurement sample, a photoelectric conversion element for converting the intensities of blank light (reference light which has transmitted through without being irradiated at the measurement sample) and light from the measurement sample into currents, and the like. This semiconductor element portion 14 generates an electrical signal corresponding to the light from the measurement sample and the blank light and the composition and structure of the measurement sample are identified on the basis of this signal.

### Embodiment 2

LAPS-equipped chip: Figure 4 is a cross-sectional view used to explain a second constitution of a microchip of the present invention, wherein the microchip shown in this figure is a chip equipped with a LAPS (Light Addressable Potentiometric Sensor) capable of detecting a surface potential (that of the SOI layer) which varies according to the amount of charge the measurement sample has.

In this figure, reference numeral 15 denotes an insulating layer formed on a surface of the SOI layer 12, reference numeral 16 denotes a sample-holding portion provided on the insulating layer 15, reference numeral 17a denotes a measurement sample, reference numeral 17b denotes a sensitive membrane, reference numerals 18a and 18b denote biasing electrodes used to form a depletion layer in a boundary face between the insulating layer 15 and the SOI layer 12, reference numeral 19 denotes a signal-detecting circuit for detecting the amount of photoelectric current generated depending on the thickness of the depletion layer which varies according to the amount of charge provided to the sensitive membrane 17b by the measurement sample, and reference numeral 60 denotes a semiconductor laser for generating electron-hole pairs within the depletion layer by means of light irradiation.

The sensor surface of this LAPS-equipped chip is the SOI layer 12 in which the insulating layer 15, such as oxide silicon, is formed, wherein a bias is applied to between the measurement sample 17a and the SOI layer 12 (substantially between the insulating layer 15 and the SOI layer 12) from the biasing electrodes 18a and 18b to form a depletion layer in a boundary face between the insulating layer 15 and the SOI layer. On the other hand, laser light from the semiconductor laser 60 is irradiated at the quartz substrate 20 from the rear surface thereof, thereby forming electron-hole pairs within the depletion layer. Under a biased environment in which a region near the boundary face between the insulating layer 15 and the SOI layer 12 is in a state of accumulation of electron-hole pairs, no photocurrents flow into an external circuit. However, if the region near the boundary face between the insulating layer 15 and the SOI layer 12 goes into an inverted state, the thickness of the depletion layer increases, thereby causing a photocurrent to flow into the external circuit.

If the amount of charge accumulated in the sensitive membrane 17b shown in Figure 4 changes, the surface potential of the SOI layer 12 also changes, thereby causing a change in the threshold of a bias voltage for the photocurrent to flow. Hence, if the amount of photoelectric current generated depending on the thickness of the depletion layer is detected by the signal-detecting circuit 19, then the amount of charge accumulated in the sensitive membrane 17b is determined from this amount of photoelectric current. For example, if a cell immersed in a culture electrolyte is mounted on the sample-holding portion 16 and electrical stimulation is applied to the cell from the outside, a potential in the cell changes and, therefore, the amount of charge to be accumulated in the sensitive membrane 17b also changes. Since this change in the charge amount is detected as a modulation of the photocurrent, it is possible to detect the surface potential of the SOI layer that varies according to the amount of charge attributable to the cell which is the measurement sample.

While aspects of the present invention have been described with reference to the embodiments thereof, it should be noted that the above-described embodiments are merely examples for carrying out the present invention and the present invention is not limited to these embodiments. Modifying these embodiments variously is within the scope of the present invention and it is obvious, from the foregoing description, that other various embodiments are possible within the scope of the present invention.

### Industrial Applicability

According to the present invention, it is possible to provide an SOI substrate having an SOI layer which has little defects and is superior in film uniformity, crystal quality, and electrical characteristics (carrier mobility and the like). In addition, use of this SOI substrate makes it possible to obtain a microchip (biochip) in which a hole, a micro-flow passage or the like and a semiconductor element for analysis/evaluation are integrated into a single chip, or a macro chip, such as a surface potential sensor, capable of monitoring a change in the charge amount of a sample (for example, cell).

## Claims

1. A method of manufacturing an SOI substrate wherein only low-temperature treatments are applied consistently at 300°C or lower comprising the following steps (1) to (4):
(1) a step of forming a hydrogen ion-implanted layer (11) by implanting ions into the bonding surface of a silicon substrate (10);
(2) a step of applying a surface activation treatment to the bonding surface of at least one of said silicon substrate (10) and a glass substrate (20);
(3) a step of bonding together said silicon substrate (11) and said glass substrate (20) and
(4) a step of transferring a silicon layer (12) onto said glass substrate (20) by peeling off the surface layer of said silicon substrate (10) along said hydrogen ion-implanted layer (11) at a temperature of 300°C or lower, and said method including a sub-step in step (3) of heat-treating said silicon substrate (10) and said glass substrate (20) after said bonding together, with said silicon substrate (10) and said glass substrate (20) bonded together, and wherein said sub-step of heat treatment is carried out at a temperature of 100°C or higher but not higher than 300°C.

2. The method according to claim 1, **characterized in that** said step (2) of surface activation treatment is carried out by means of at least one of plasma treatment and ozone treatment.

3. The method according to claim 1 or 2, **characterized in that** said method includes the following step in succession to said step (4):
(5) a step of polishing the peeling plane of said silicon layer so that the surface roughness (RMS) thereof is not greater than 3 nm.

4. The method according to any one of claims 1 to 3, **characterized in that** one principal surface of said glass substrate has a concave portion, such as a flow passage or a hole, and a semiconductor element for analyzing/evaluating a sample attached/held to said concave portion is provided in said silicon layer provided on the other principal surface of said glass substrate.

5. The method according to any one of claims 1 to 4, **characterized by** including an insulating layer formed on a surface of said silicon layer; sample-holding means provided on said insulating layer; biasing means for forming a depletion layer in a boundary face between said insulating layer and said silicon layer; and a signal-detecting circuit for detecting the amount of photoelectric current generated depending on the thickness of said depletion layer which varies according to the amount of charge provided by an analyte held by said sample-holding portion.

6. The method according to any one of claims 1 to 5, **characterized in that** said glass substrate is a quartz substrate.

## Patentansprüche

1. Verfahren zur Herstellung eines SOI-Substrats, in dem ausschließlich Niedrigtemperaturbehandlungen konsistent bei 300 °C oder darunter angewendet werden, umfassend die folgenden Schritte (1) bis (4):
(1) einen Schritt der Bildung einer Wasserstoff-lonen-implantierten Schicht (11) durch Implantieren von Ionen in die Bindungsoberfläche eines Siliciumsubstrats (10);
(2) einen Schritt der Anwendung einer Oberflächenaktivierungsbehandlung auf die Bindungsoberfläche von mindestens einer des Siliciumsubstrats (10) und des Glassubstrats (20);
(3) einen Schritt der Verbindung des Siliciumsubstrats (11) mit dem Glassubstrat (20), und
(4) einen Schritt der Transferierung einer Siliciumschicht (12) auf das Glassubstrat (20) durch Abziehen der Oberflächenschicht des Siliciumsubstrats (10) entlang der Wasserstoff-lonen-implantierten Schicht (11), bei einer Temperatur von 300 °C oder geringer, und das Verfahren umfasst einen Unterschritt in Schritt (3) der Wärmebehandlung des Siliciumsubstrats (10) und des Glassubstrats (20) nach deren Verbindung, wobei das Siliciumsubstrat (10) und das Glassubstrat (20) miteinander verbunden werden, und wobei der Unterschritt der Wärmebehandlung ausgeführt wird bei einer Temperatur von 100 °C oder darüber, aber nicht größer als 300 °C.

2. Verfahren nach Anspruch 1, **dadurch gekennzeichnet, dass** der Schritt (2) der Oberflächenaktivierungsbehandlung durchgeführt wird durch mindestens eine von Plasma-Behandlung und Ozon-Behandlung.

3. Verfahren nach Anspruch 1 oder 2, **dadurch gekennzeichnet, dass** das Verfahren im Anschluss an Schritt (4) den folgenden Schritt einschließt:
(5) einen Schritt des Polierens der Abziehebene der Siliciumschicht, so dass die Oberflächenrauheit (RMS) davon nicht größer als 3 nm ist.

4. Verfahren nach irgendeinem der Ansprüche 1 bis 3, **dadurch gekennzeichnet, dass** eine Hauptoberfläche des Glassubstrats einen konkaven Bereich hat, wie beispielsweise eine Fließpassage oder ein Loch, und ein Halbleiterelement für die Analyse/Bewertung einer Probe, die an dem konkaven Bereich angebracht oder gehalten wird, vorgesehen ist in der Siliciumschicht, die auf der anderen Hauptoberfläche des Glassubstrats vorgesehen ist.

5. Verfahren nach irgendeinem der Ansprüche 1 bis 4, **dadurch gekennzeichnet, dass** eingeschlossen sind eine Isolierschicht, gebildet auf einer Oberfläche der Siliciumschicht; Probenhaltemittel, die bereitgestellt werden auf der Isolierschicht; Vorspannmittel zur Bildung einer Abbauschicht in einer Grenzfläche zwischen der Isolierschicht und der Siliciumschicht; und ein Signal-aufzeichnender Stromkreis zur Aufzeichnung einer photoelektrischen Strommenge, die in Abhängigkeit von der Dicke der Abbauschicht erzeugt wird, die entsprechend der Ladungsmenge, die durch einen Analyten bereitgestellt wird, der durch den Probenhaltbereich gehalten wird, variiert.

6. Verfahren nach irgendeinem der Ansprüche 1 bis 5, **dadurch gekennzeichnet, dass** das Glassubstrat ein Quartzsubstrat ist.

## Revendications

1. Procédé de fabrication d'un substrat de SOI dans lequel seuls des traitements à basse température sont appliqués de façon constante à 300 °C ou moins, comprenant les étapes (1) à (4) suivantes :
(1) une étape de formation d'une couche à ions hydrogène implantés (11) par implantation d'ions dans la surface de liaison d'un substrat en silicium (10) ;
(2) une étape d'application d'un traitement d'activation de surface à la surface de liaison dudit substrat de silicium (10) et/ou d'un substrat de verre (20) ;
(3) une étape de liaison ensemble dudit substrat de silicium (11) et dudit substrat de verre (20) ; et
(4) une étape de transfert d'une couche de silicium (12) sur ledit substrat de verre (20) par décollement de la couche de surface dudit substrat de silicium (10) le long de ladite couche à ions hydrogène implantés (11) à une température de 300 °C ou moins, et ledit procédé comprenant une sous-étape dans l'étape (3) de traitement thermique dudit substrat de silicium (10) et dudit substrat de verre (20) après ladite liaison ensemble, avec ledit substrat de silicium (10) et ledit substrat de verre (20) collées ensemble, et dans laquelle ladite sous-étape de traitement thermique est effectuée à une température de 100 °C ou plus mais pas supérieure à 300 °C.

2. Procédé selon la revendication 1, **caractérisé en ce que** ladite étape (2) de traitement d'activation de surface est réalisée au moyen d'un traitement au plasma et/ou d'un traitement à l'ozone.

3. Procédé selon la revendication 1 ou 2, **caractérisé en ce que** ledit procédé comprend l'étape suivante, successive à ladite étape (4) :
(5) une étape de polissage du plan de décollement de ladite couche de silicium de sorte que sa rugosité de surface (RMS) ne soit pas supérieure à 3 nm.

4. Procédé selon l'une quelconque des revendications 1 à 3, **caractérisé en ce qu'**une surface principale dudit substrat de verre comporte une partie concave, telle qu'un passage d'écoulement ou un trou, et un élément semi-conducteur pour analyser/évaluer un échantillon fixé/maintenu à ladite partie concave est disposé dans ladite couche de silicium disposée sur l'autre surface principale dudit substrat de verre.

5. Procédé selon l'une quelconque des revendications 1 à 4, **caractérisé en ce qu'**il comprend une couche isolante formée sur une surface de ladite couche de silicium ; des moyens de maintien d'échantillon disposés sur ladite couche isolante ; des moyens de polarisation pour former une couche d'appauvrissement dans une face limite entre ladite couche isolante et ladite couche de silicium ; et un circuit de détection de signal pour détecter la quantité de courant photoélectrique produit en fonction de l'épaisseur de ladite couche d'appauvrissement qui varie en fonction de la quantité de charge fournie par un analyte maintenu par ladite partie de maintien d'échantillon.

6. Procédé selon l'une quelconque des revendications 1 à 5, **caractérisé en ce que** ledit substrat de verre est un substrat de quartz.
